# EUROPÄISCHE PATENTSCHRIFT

(11) **EP 0 521 388 B1**
(45) Veröffentlichungstag und Bekanntmachung des Hinweises auf die Patenterteilung: **10.05.1995**
(21) Anmeldenummer: 92110679.5
(22) Anmeldetag: 25.06.1992
(51) Int. Cl.: A61K 9/00, A61K 9/46

(54) **Verfahren zur Herstellung einer pharmazeutischen Zubereitung mit wenigstens zwei verschiedenen Wirkstoffen und Verwendung einer solchen Zubereitung**
Process for the preparation of a pharmaceutical preparation containing at least two different actives and use thereof
Procédé de préparation d'une composition pharmaceutique à base d'au mains deux principes actifs différents et utilisation d'une telle composition

(30) Priorität: 01.07.1991 CH 1944/91
(43) Veröffentlichungstag der Anmeldung: 07.01.1993
(73) Patentinhaber: Gergely, Gerhard, Dr., A-1053 Wien (AT)
(72) Erfinder: Gergely, Gerhard, Dr., A-1053 Wien (AT); Tritthart, Wolfram, Dr., A-9400 Wolfsberg (AT)
(74) Vertreter: Büchel, Kurt F., Dr.

(56) Entgegenhaltungen:
- EP-A- 0 418 564
- AT-A- 373 492
- BE-A- 666 198
- DE-A- 2 743 702

## Beschreibung

Die therapeutische Behandlung verschiedener Krankheitszustände bedarf - insbesondere bei älteren Menschen - sehr häufig einer manchmal über Wochen oder Monate, ja sogar Jahre hinausgehenden Einnahme verschiedener pharmazeutischer Wirkstoffe, von denen einige täglich oder sogar mehrmals täglich, andere vielleicht nur jeden zweiten Tag oder gar nur einmal pro Woche einzunehmen sind. Dazu kommt, dass die Dosierung sehr oft für einen bestimmten Patienten vom Arzt individuell eingestellt und mit fortschreitender Zeit variiert werden soll. Dies ist mit den herkömmlichen Darreichungsformen von Tabletten oder Dragees nur schwer zu bewerkstelligen. Einerseits wird nämlich die Einnahme von Tabletten - wiederum insbesondere bei älteren Menschen, die häufig an Schluckbeschwerden leiden - als unangenehm empfunden, insbesondere wenn eine Vielzahl solcher Tabletten - manchmal zu jeder Mahlzeit - einzunehmen ist; andererseits ist die Variation der Dosierung kompliziert: kleine Dragees oder Tabletten sind vom Patienten nur schwer zu teilen, wenn z.B. halbe oder Viertel-Tabletten verabreicht werden sollen.

Die Erfindung hat sich daher die Aufgabe gestellt, eine Darreichungsform für zwei oder mehr Wirkstoffe zu schaffen, die auch älteren Patienten und/oder solchen mit Schluckbeschwerden leichtes Einnehmen ermöglicht, und die andererseits dem Arzt sehr weitgehende Freiheit bei der individuellen Dosierung und ihrer Variation über die Fortdauer einer medikamentösen Behandlung belässt. Diese Aufgabe wird durch die Merkmale des Anspruchs 1 überraschend und zufriedenstellend gelöst. Weiterbildungen der Erfindung sind in den Kennzeichen der abhängigen Ansprüche beschrieben.

Man hat bisher immmer nur **eine** Brausetablette in Wasser gelöst. Der Grund dafür war einerseits, dass die Brausetabletten zur vollständigen Auflösung ein Gewicht von 2 g oder mehr, meist 3 bis 4 g hatten, und daher mindestens 100, eher 200 bis 250 ml Wasser zur Auflösung brauchten. Nun nehmen jedoch viele - insbesondere ältere - Menschen ungern so grosse Flüssigkeitsmengen zu sich, notabene wenn sie dann zwei oder gar mehrere solcher Tabletten einzunehmen hätten.

Andererseits würde eine individuelle Dosierung, insbesondere z.B. nach einem Stufenplan, wie er gerne zur Behandlung der Hypertonie verwendet wird, eine Vielzahl solcher Mischungen wechselnder Zusammensetzung benötigen, was sowohl für die Herstellung als auch für den Vertrieb eine unzumutbare Belastung bedeuten würde.

Eine für jeden Fachmann selbstverständliche Voraussetzung bei der Herstellung von Mischungen zur Durchführung des erfindungsgemässen Verfahrens ist die Auswahl solcher Wirkstoffe oder Wirkstoff-Formen, bzw. solcher Brause-, Lös- und/oder Zerfallssysteme, die in wässriger Lösung zumindest kurzzeitig miteinander kompatibel sind. Die Tatsache, dass diese Kompatibilität nur kurzzeitig gegeben sein muss, ist ein weiterer Vorteil der vorliegenden Erfindung; man kann daher durchaus Verbindungen bzw. Systeme wählen, die bei längerer Lagerung bzw. bei längerer Aufbewahrung in Lösung (z.B. in Trinkampullen) miteinander in unerwünschter Weise reagieren würden und daher in dieser Form nicht zur Einnahme durch den Patienten geeignet sind. Viele solcher Verbindungen bzw. Systeme sind aber in wässriger Lösung die wenigen Sekunden oder Minuten, die allenfalls zwischen Zubereitung der Lösung oder Suspension nach dem erfindungsgemässen Verfahren und der Einnahme durch den Patienten verstreichen, ausreichend stabil, weil sie - insbesondere bei Raumtemperatur - erst nach längerer Zeit hydrolisieren oder anderweitig miteinander reagieren.

Insbesondere bei der Behandlung der Herzinsuffizienz und/oder der Hypertonie, zur individuellen Einstellung eines Patienten auf die tatsächlich benötigte Dosierung und Verteilung dieser Dosierung der benötigten Medikamente über den Zeitverlauf, hat sich das erfindungsgemässe Verfahren bewährt. Bei dieser Behandlung ist nämlich ganz besonders stark mit verschiedenen Medikamenten in verschiedener Dosierung zu operieren, wobei die Dosierung dann gelegentlich im Verlauf der Behandlung- manchmal sogar in kürzeren Zeitabständen - anzupassen, das heisst, zu ändern ist. Insbesondere hier bietet das erfindungsgemässe Verfahren daher einen beträchtlichen Fortschritt.

Weniger dringlich, wenngleich auch nicht ohne Vorteil, ist die Anwendung des erfindungsgemässen Verfahrens zur Behebung von Mangelerscheinungen, wo die Dosierung oft unproblematisch oder zumindestens nicht kritisch ist und daher den Patienten selbst überlassen werden kann, z.B. im Rahmen der sogenannten OTC-Präparate, wie sie z.B. für Vitamin-, Calcium-, Magnesium- oder Spurenelement-Gaben üblich sind.

Zweckmässig ist auch die Anwendung des erfindungsgemässen Verfahrens bei der Behandlung von Asthma, indem z.B. Antiallergika, z.B. Ketotifen, mit Sekretolytika oder Mukolytika wie etwa Acetylcystein, Bromhexin, Ambroxol oder mit oralen Betaminetika wie beispielsweise Terbutalin oder Salbutamol kombiniert werden.

Ein weiteres Anwendungsgebiet liegt in der Ulcus-Therapie durch Kombination von H2-Antagonisten, z.B. Cimetidin, Ranitidin mit Wismutpräparaten und/oder Sucralfat. In diesem Fall ist besonders die kombinierte Behandlung interessant, weil etwa Wismutpräparate über einen Zeitraum von 2 bis 4 Wochen zur Behandlung des Campylobacter Pylori gegeben werden, während die H2-Blocker zur Verringerung der Säuresekretion über 6 bis 8 Wochen verabreicht werden. Eine Kombination von H2-Blockern mit Sucralfat in der Akutphase wäre ebenso sinnvoll. Bei Absetzen von H2-Blockern ist ein massives Ansteigen der Säuresekretion oft mit der Ausbildung von Rezidiven verbunden. Daher ist es oft notwendig, eine Rezidivprophylaxe mit niedrigen Dosen (z.B. halben Tabletten) durchzuführen.

Schliesslich ist auch die Behandlung oder Vorbeugung der Osteoporose ein bevorzugtes Anwendungsgebiet des erfindungsgemässen Verfahrens, z.B. durch Kombination von Calciumpräparaten mit Fluorpräparaten, z.B. Natriumfluorophosphat und/oder Diphosphonaten. Besonders in der Therapie mit Diphosphonaten, z.B. Etidronat, wird eine intermittierende Behandlung gleichzeitig mit Calciumpräparaten durchgeführt. Derartige Behandlungsschemata werden auch mit Natriumfluorophosphat kombiniert. Insbesondere die Intervallbehandlung mit Lös- bzw. Zerfallstabletten ist für solche Kombinationen zweckmässig. Die Kombination von Calciumpräparaten mit Östrogen bzw. Östrogen-Kombinationspräparaten ist wiederum für die Osteoporose-Prophylaxe zu empfehlen.

Für alle genannten Beispiele gilt, dass die Bereitung eines "Cocktails" aus verschiedenen Wirkstoffen in Form von Lös- bzw. Zerfallstabletten für den Patienten eine Einnahme bedeutet. Der "Cocktail" erlaubt eine individuelle Dosierung sowie eine intermittierende Behandlung und eröffnet somit gute therapeutische Möglichkeiten.

### Beispiel 1:

Ein 60-jähriger Patient zeigt Hypertonie mit Anzeichen einer Herzinsuffizienz. Es werden als Dauertherapie täglich 20 mg ACE-Hemmer (z.B. Enalapril) in einschleichender Dosierung über wenige Tage und 20 mg Furosemid verabreicht. Sollte das nicht innerhalb weniger Tage ausreichen, werden 1 x 20 mg oder 2 x 10 mg Calcium-Antagonist (z.B. Nifedipin) pro Tag gegeben. Sollte der Patient allerdings Krampfadern haben, ist der Calcium-Antagonist durch eine Erhöhung der Dosis des ACE-Hemmers zu ersetzen.

### Beispiel 2:

Ein Patient mit Hypertonie und angedeuteter Herzinsuffizienz bekommt täglich 20 mg Nifedipin. Bei bestehenden Krampfadern sind Beinödeme aufgetreten. Aus diesem Grund erhält er zusätzlich Furosemid, beginnend mit 20 mg/d; die Dosis mag im Verlauf der Behandlung auf 30-40 mg/d gesteigert werden, insbesondere wenn die Anzeichen der Herzinsuffizienz zunehmen sollten (Staulunge, Beinschwellungen).

### Beispiel 3:

Ein 45-jähriger Patient mit 85 kg Körpergewicht zeigt chronische Hypertonie seit 10 Jahren. Man verabreicht ihm z.B. 100 mg Atenolol und 25 mg Hydrochlorothiazid täglich; wenn es dem Patienten gelingt, 10 kg abzunehmen, kann die Atenolol-Dosis auf 50 mg täglich reduziert werden. Sollte sich bei der Therapie mit dem Beta-Blocker der Puls zu stark verlangsamen, wird der Beta-Blocker durch einen Calcium-Antagonisten ersetzt. Sollte die Hypertonie innerhalb weniger Tage nicht abnehmen, muss die Beta-Blocker-Dosis entsprechend erhöht werden.

### Beispiel 4:

Ein Patient mit chronisch-asthmoider Bronchitis erhält zunächst 3 x 25 mg Salbutamol und 3 x 30 mg Ambroxol pro Tag. Die Dosen werden je nach Fortschritt der Heilung reduziert.

### Beispiel 5:

Eine 55-jährige Frau mit Wechselbeschwerden und/oder Anzeichen beginnender Osteoporose erhält zunächst 50 mg Östradiol und 1000 mg eines geeigneten Calziumpräparates pro Tag, welch letzteres bei vernünftiger Ernährung bald auf 500 mg täglich reduziert werden kann.

### Beispiel 6:

61-jähriger Patient leidet seit 10 Jahren an Diabetes mell. und seit ca. 5 Jahren an einer Hypertonie.
Bisher mit Metoprolol in Retardform (Beloc Duriles) als Metoprololtartrat 200 mg und Glibenclamid (morgens 5 mg) relativ gut eingestellt.

Nach Auftreten einer schweren, akuten, obstruktiven Bronchitis notwendige Umstellung auf Enalapril 10 mg morgens und wegen Verschlechterung der diabet. Stoffwechsellage Steigerung von Glibenclamid auf morgens und abends je 5 mg. Ausserdem zusätzliche Gabe von Terbutalin 0,25 mg als Dosieraerosol 3 x täglich 2 Hübe und Acetylcystein 2 x 100 mg.
Danach deutliche Besserung der Obstruktion und gute Einstellung von Blutdruck und Diabetes.

### Beispiel 7:

64-jährige Patientin seit 7 Jahren wegen bestehender Hypertonie mit Lisinopril 5 mg gut eingestellt. Wegen zunehmender Übelkeit und Durchfällen Durchuntersuchung, wobei in den Laborwerten deutlich erhöhtes Kreatinin und eine Hyperkaliämie festgestellt wurden. Sofortiges Absetzen von Lisinopril und neben strenger Überwachung und Infusionstherapie Übergang auf Nifedipin 2 x 10 mg sowie Furosemid 40 mg morgens. Daraufhin nach verstärkter Diurese deutlicher Rückgang der Nierenparameter sowie der Hyperkaliämie.

### Beispiel 8:

56-jähriger Patient leidet an einer koronaren Herzkrankheit (vor 5 Jahren Infarkt), sowie seit 3 Jahren an Hypertonus. Seither mit Nifedipin 2 x 20 mg und Atenolol 50 mg morgens gut eingestellt. Plötzlich Entgleisung des Blutdruckes mit systolischen Werten über 200, zusätzliche Gabe von 25 mg Hydrochlorothiazid 3 x wöchentlich, trotzdem keine ideale Einstellung.
Hierauf Umstellung auf Captopril 3 x 12,5 mg und Atenolol 50 mg + Hydrochlorothiazid 3 x 25 mg pro Woche. Daraufhin befriedigende RR-Werte systolisch zwischen 140 und 160, diastolisch zwischen 80 und 95 mm Hg.

### Beispiel 9:

28-jährige Frau seit 10 Jahren an einem exogen-allergischen Asthma bronchiale erkrankt. Nimmt seither Ketotifen 2 x 1 mg, fallweise 2 x 2 mg täglich. Nach einem akuten Infekt der oberen Luftwege Verschlechterung der Obstruktion.
Daraufhin zusätzliche Gabe von Erythromycin 3 x 500 mg sowie Acetylcystein 2 x 100 mg. Nach 3 Tagen Entwicklung eines allergischen Exanthems, so dass nochmalige Umstellung auf Doxycyclin 100 mg 2 x täglich sowie Gabe von Ambroxol 75 mg 2 x täglich.
Abklingen des Exanthems, Besserung der Obstruktion und des Infekts.

### Beispiel 10:

75-jähriger Patient leidet an schwerer Osteoporose, deshalb mit Fluor-Calcium-Präparat seit Jahren behandelt. Zusätzlich wegen Magenbeschwerden (keine Endoskopie oder Röntgen) Sucralfat 2x2g.
Nach akuter Verschlechterung der Kreuzschmerzen Gabe von Diclofenac 2 x 50 mg, worauf die Magenbeschwerden zunahmen. Die Gastroskopie ergab ein Ulcus duodeni. Nach Absetzen von Diclofenac und zusätzlicher Gabe von Cimetidin 800 mg plus abends zum Sucralfat Verschwinden der Magenbeschwerden.
Die Osteoporose wird mit Etidronat 2 x 200 mg im Intervall zusätzlich behandelt.

### Beispiel 11:

32-jähriger Patient, recidivierender Ulcus duodeni. Bisher mit Cimetidin 800 mg abends und zusätzlich Sucralfat immer wieder behandelt.
Gastroskopie und Biopsie ergaben einen positiven Befund. Daraufhin Gabe von Bismutnitrat-oxid 3 x 350 mg über 4 Wochen. Gastroskopiekontrolle ergab abgeheilten Ulcus. Weitere Kontrolle nach einem Jahr --> ohne Ergebnis!

### Beispiel 12:

53-jährige, grazile, blonde Frau ist seit dem 49. Lebensjahr im Klimakterium.
Anlässlich einer gynäkologischen Kontrolle wird ihr eine Densitometrie der Wirbelkörper empfohlen, die eine beginnende Osteoporose ergab. Daraufhin empfiehlt ihr der Arzt eine Prophylaxe mit einem Östrogen-Gestagen Präparat und Calcium 500 mg täglich.

### Beispiel 13:

35-jähriger Patient nimmt wegen Herzrhythmusstörungen (super-ventrik. und ventrik. Extrasystolen) seit 1 Monat Metaprolol 2 x 50 mg und Magnosolv, seither leidet er unter Schlafstörungen und Depressionen. Das Weglassen der Abenddosis vermindert zwar diese Beschwerden, dafür treten nachts wieder vermehrt Rhythmusstörungen auf. Nach Wechsel des β-Blockers (vom lipophilen Metaprolol zum hydrophilen Atenolol) sind diese Beschwerden verschwunden.

### Beispiel 14:

47-jähriger mit chron. obstruktiver Bronchitis steht seit Jahren unter Clenbuterol 0,02 mg bei Bedarf. Wegen Verschlechterung Steigerung der Dosis auf 3 x täglich 0,02 mg. Zusätzlicher Infekt (bakteriell) und daher Therapie mit Cotrimoxazol 2 x 1 Tablette und wegen zähem Schleim Bromhexin 8 mg 3 x täglich mit viel Flüssigkeit. Danach rasche Besserung sowohl des Auswurfes als auch der Atemnot.

Um die in den vorstehenden Beispielen verschiedentlich angeführten Wirkstoffe zu verabreichen, werden erfindungsgemäss Tabletten aus folgenden Zusammensetzungen hergestellt:

### Acetylcystein Löstablette:

200 mg Acetylcystein
320 mg Mannitol
342 mg Zitronensäure
160 mg Weinsäure
287 mg Natriumhydrogencarbonat
33 mg Polyvinylpyrrolidon
20 mg Aroma
10 mg Aspartame

### Östrogen/Medroxyprogestron Zerfallsbrause:

2,0 mg Östradiolvalerianat
20,0 mg Medroxyprogesteron-acetat
33,0 mg Polyvinylpyrrolidon
25,0 mg Polyvinylpyrrolidon XL
350,0 mg Zitronensäure
280,0 mg Natriumhydrogencarbonat
0,2 mg Dioctylsulfosuccinat
167,0 mg Mannitol

### Calcium Brausetablette:

2.500 mg Calciumcarbonat
4.270 mg Zitronensäure
150 mg Gluconsäure-Deltalacton
8 mg Saccharin-Natrium
50 mg Aroma
27 mg Polyethylenglycol

### Östrogen-Calcium gegen Osteoporose mit Auslösung einer Abbruchsblutung:

70 Tage Calcium Brausetablette + Östrogen Löstablette
2 Wochen Calcium + Östrogen/Medroxyprogesteron
1 Woche nur Calcium

### Östrogen Löstablette:

2 mg Östradiolvalerianat
7 mg polyvinylpyrrolidon
33 mg Lactose
426 mg Zitronensäure
315 mg Natriumhydrogencarbonat
25 mg Kaliumcarbonat

### Salbutamol Löstablette:

2,8 mg Salbutamolsulfat
425,0 mg Zitronensäure
10,0 mg Natriumcitrat
286,0 mg Natriumhydrogencarbonat
40,0 mg Natriumcarbonat
67,0 mg Mannitol
4,0 mg Saccharin-Natrium
15,0 mg Aroma
Die Standarddosierung beträgt peroral 2,8 mg Salbutamolsulfat, entsprechend 2 mg Salbutamol.

### Enalapril Löstablette:

20 mg Enalapril
142 mg Mannitol
710 mg Zitronensäure
86 mg Weinsäure
290 mg Natriumhydrogencarbonat
50 mg Natriumcarbonat
4 mg Saccharin-Natrium
20 mg Aroma
Die Tablette ist mit einer Bruchrille versehen, so dass exakt 10 mg, 20 mg oder auch die doppelte Dosis 20 mg (= 2 Tabletten) zur Cocktail-Bereitung verwendet werden.

### Nifedipin Zerfallstablette:

10 mg Nifedipin
20 mg Maisstärke
28 mg Polyvinylpyrrolidon (quervernetzt)
70 mg Polyvinylpyrrolidon
350 mg Natriumhydrogencarbonat
410 mg Zitronensäure
42 mg Avicel

### Furosemid Zerfallsbrause:

40,00 mg Furosemid
36,00 mg Maisstärke
20,00 mg Polyvinylpyrrolidon (quervernetzt)
720,00 mg Weinsäure
375,00 mg Natriumhydrogencarbonat
15,00 mg Aspartame
20,00 mg Aroma
0,15 mg Natriumdioctylsulfosuccinat
Eine Bruchrille erlaubt die Einzeldosierung von 20 mg Furosemid.

### Atenolol Brausetablette:

200 mg Atenolol
510 mg Zitronensäure
40 mg Fumarsäure
2 mg Natriumcitrat
100 mg Kaliumhydrogencarbonat
243 mg Natriumhydrogencarbonat
45 mg Mannitol
62 mg Lactose
10 mg Aspartame
15 mg Aroma
Eine Bruchrille erlaubt die individuelle Dosierung. Angina pectoris: Dosis 50-400 mg/Tag.

### Atenolol Löstablette:

100 mg Atenolol
43 mg Apfelsäure
291 mg Zitronensäure
220 mg Natriumhydrogencarbonat
37 mg Kaliumhydrogencarbonat
50 mg Magnesiumcarbonat
86 mg Mannitol
5 mg Aspartame
10 mg Aroma
Eine Bruchrille ermöglicht die individuelle Dosierung.
- Hypertension:: Dosis 50-200 mg/Tag
- Angina pectoris:: Dosis 50-400 mg/Tag.

### Ambroxol Löstablette:

33 mg Ambroxol-Hydrochlorid
470 mg Weinsäure
34 mg Kaliumcarbonat
310 mg Natriumhydrogencarbonat
45 mg Glucose
4 mg Saccharin-Natrium
15 mg Aroma

### Sucralfat Dispersions-Granulat:

2.000 mg Sucralfat
1.130 mg Mannitol
115 mg Avicel
86 mg Polyvinylpyrrolidon XL
23 mg Polyvinylpyrrolidon
30 mg Aspartame
20 mg Aroma

### Bismutnitrat Löstablette:

350 mg Bismutnitrat
1.320 mg Weinsäure
54 mg Lactose
131 mg Mannitol
870 mg Natriumhydrogencarbonat
3 mg Saccharin-Natrium
28 mg Natriumcyclamat
30 mg Aroma

### Ranitidin Löstablette:

200 mg Ranitidin
430 mg Zitronensäure
38 mg Kaliumhydrogencarbonat
290 mg Natriumhydrogencarbonat
37 mg Natriumcarbonat
15 mg Aspartame
10 mg Aroma

## Patentansprüche

1. Verfahren zur Herstellung einer wässrigen Lösung oder Dispension zur oralen Einnahme von wenigstens zwei verschiedenen pharmazeutischen Wirkstoffen, dadurch gekennzeichnet, dass wenigstens zwei verschiedene Brause-, Lös- und/oder Zerfallsmischungen in Tabletten- bzw. Granulatform, von denen jede einen unterschiedlichen Wirkstoff oder eine unterschiedliche Wirkstoffkombination enthält, in ein und derselben Wassermenge aufgelöst bzw. suspendiert werden.

2. Verfahren nach Anspruch 1, dadurch gekennzeichnet, dass wenigstens eine der Tabletten nur als Bruchstück eingesetzt wird.

3. Verfahren nach Anspruch 1 oder 2, dadurch gekennzeichnet, dass wenigstens eine der Mischungen weniger als 2000, vorzugsweise weniger als 1500, insbesondere weniger als 1000 mg wiegt.

4. Verfahren nach einem der vorhergehenden Ansprüche, dadurch gekennzeichnet, dass wenigstens eine der Mischungen ein Diuretikum, und wenigstens eine der weiteren Mischungen einen Beta-Blocker und/oder einen Vasodilatator enthält.

5. Verfahren nach Anspruch 4, dadurch gekennzeichnet, dass als Diuretikum Hydrochlorothiazid und/oder Furosemid eingesetzt werden.

6. Verfahren nach Anspruch 4, dadurch gekennzeichnet, dass als Vasodilatator wenigstens eine der folgenden Substanzen eingesetzt wird: Naftidrofuryl, Calcium-Antagonist, ACE-Hemmer, Alpha-1-Blocker.

7. Verwendung von wenigstens zwei verschiedenen Brause-, Lös- und/oder Zerfallsmischungen in Tabletten- bzw. Granulatform, von denen jede einen unterschiedlichen Wirkstoff oder eine unterschiedliche Wirkstoffkombination enthält, zur Herstellung einer einzigen wässrigen Lösung oder Suspension eines Diuretikums mit wenigstens einem Beta-Blocker und/oder Vasodilatator für die Behandlung der Hypertonie.

8. Verwendung von wenigstens zwei verschiedenen Brause-, Lös- und/oder Zerfallsmischungen in Tabletten- bzw. Granulatform, von denen jede einen unterschiedlichen Wirkstoff oder eine unterschiedliche Wirkstoffkombination enthält, zur Herstellung einer einzigen wässrigen Lösung oder Suspension eines Antiallergikums mit wenigstens einer der Substanzen Sekretolytikum, Mukolytikum und Betaminetikum zur Behandlung von Asthma.

9. Verwendung von wenigstens zwei verschiedenen Brause-, Lös- und/oder Zerfallsmischungen in Tabletten- bzw. Granulatform, von denen jede einen unterschiedlichen Wirkstoff oder eine unterschiedliche Wirkstoffkombination enthält, zur Herstellung einer einzigen wässrigen Lösung oder Suspension eines H2-Antagonisten mit wenigstens einem Wismutsalz und/oder Sucralfat für die Ulcus-Therapie.

10. Verwendung von wenigstens zwei verschiedenen Brause-, Lös- und/oder Zerfallsmischungen in Tabletten- bzw. Granulatform, von denen jede einen unterschiedlichen Wirkstoff oder eine unterschiedliche Wirkstoffkombination enthält, zur Herstellung einer einzigen wässrigen Lösung oder Suspension eines Calciumsalzes mit wenigstens einem Fluorsalz und/oder Diphosphonat für die Behandlung der Osteoporose.

11. Verwendung von wenigstens zwei verschiedenen Brause-, Lös- und/oder Zerfallsmischungen in Tabletten- bzw. Granulatform, von denen jede einen unterschiedlichen Wirkstoff oder eine unterschiedliche Wirkstoffkombination enthält, zur Herstellung einer einzigen wässrigen Lösung oder Suspension eines Calciumsalzes mit wenigstens einem Östrogenpräparat für die Vorbeugung der Osteoporose.

## Claims

1. A process for the preparation of an aqueous solution or dispersion for oral administration of at least two different pharmaceutical active ingredients, characterized in that at least two different effervescent, soluble and/or disintegrating mixtures in tablet or granular form, each of which contains a different active ingredient or a different active ingredient combination, are dissolved or suspended in one and the same quantity of water.

2. Process according to Claim 1, characterized in that at least one of the tablets is used only as a fragment.

3. Process according to Claim 1 or 2, characterized in that at least one of the mixtures weighs less than 2000, preferably less than 1500, in particular less than 1000, mg.

4. Process according to any of the preceding Claims, characterized in that at least one of the mixtures contains a diuretic, and at least one of the further mixtures contains a beta-blocker and/or a vasodilator.

5. Process according to Claim 4, characterized in that hydrochlorothiazide and/or furosimide are used as the diuretic.

6. Process according to Claim 4, characterized in that at least one of the following substances is used as the vasodilator: naftidrofuryl, a calcium antagonist, an ACE inhibitor or an alpha-1-blocker.

7. Use of at least two different effervescent, soluble and/or disintegrating mixtures in tablet or granular form, each of which contains a different active ingredient or a different active ingredient combination, for the preparation of a single aqueous solution or suspension of a diuretic with at least one beta-blocker and/or vasodilator for the treatment of hypertension.

8. Use of at least two different effervescent, soluble and/or disintegrating mixtures in tablet or granular form, each of which contains a different active ingredient or a different active ingredient combination, for the preparation of a single aqueous solution or suspension of an antiallergic agent with at least one of the substances from a secretolytic agent, a mucolytic agent and a beta-mimetic agent for the treatment of asthma.

9. Use of at least two different effervescent, soluble and/or disintegrating mixtures in tablet or granular form, each of which contains a different active ingredient or a different active ingredient combination, for the preparation of a single aqueous solution or suspension of an H2-antagonist with at least one bismuth salt and/or sucralfate for ulcer therapy.

10. Use of at least two different effervescent, soluble and/or disintegrating mixtures in tablet or granular form, each of which contains a different active ingredient or a different active ingredient combination, for the preparation of a single aqueous solution or suspension of a calcium salt with at least one fluorine salt and/or diphosphonate for the treatment of osteoporosis.

11. Use of at least two different effervescent, soluble and/or disintegrating mixtures in tablet or granular form, each of which contains a different active ingredient or a different active ingredient combination, for the preparation of a single aqueous solution or suspension of a calcium salt with at least one oestrogen preparation for the prevention of osteoporosis.

## Revendications

1. Procédé de préparation d'une solution ou dispersion aqueuse pour la prise orale d'au moins deux substances actives différentes, caractérisé en ce qu'on dissout ou met en suspension deux mélanges effervescents, solubles ou désintégrables différents sous forme de comprimés ou de granulés, dont chacun contient une substance active différente ou une combinaison de substances actives différente, dans une seule et même quantité d'eau.

2. Procédé selon la revendication 1, caractérisé en ce qu'on utilise au moins un des comprimés uniquement sous forme de fragment.

3. Procédé selon les revendications 1 ou 2, caractérisé en ce qu'au moins un des mélanges pèse moins de 2000, de préférence moins de 1500, en particulier moins de 1000 mg.

4. Procédé selon l'une des revendications précédentes, caractérisé en ce qu'au moins un des mélanges contient un diurétique, et au moins un des autres mélanges contient un bêta-bloquant et/ou un vasodilatateur.

5. Procédé selon la revendication 4, caractérisé en ce qu'on utilise comme diurétique l'hydrochlorothiazide et/ou le furosemide.

6. Procédé selon la revendication 4, caractérisé en ce qu'on utilise comme vasodilatateur au moins une des substances suivantes : le naftidrofuryl, un antagoniste du calcium, un inhibiteur de l'ACE, un alpha-1-bloquant.

7. Utilisation d'au moins deux mélanges effervescents, solubles et/ou désintégrables différents sous forme de comprimés ou de granulés, dont chacun contient une substance active différente ou une combinaison de substances actives différente, pour la préparation d'une solution ou suspension aqueuse unique d'un diurétique avec au moins un bêta-bloquant et/ou un vasodilatateur pour le traitement de l'hypertonie.

8. Utilisation d'au moins deux mélanges effervescents, solubles et/ou désintégrables sous forme de comprimés ou de granulés, dont chacun contient une substance active différente ou une combinaison de substances actives différente, pour la préparation d'une solution ou suspension aqueuse unique d'un anti-allergique avec au moins une substance secrétolytique, mucolytique et bêta-mimétique pour le traitement de l'asthme.

9. Utilisation d'au moins deux mélanges effervescents, solubles et/ou désintégrables sous forme de comprimés ou de granulés, dont chacun contient une substance active différente ou une combinaison de substances actives différente, pour la préparation d'une solution ou suspension aqueuse unique d'un antagoniste de H₂ avec au moins un sel de bismuth et/ou du Sucralfate pour le traitement des ulcères.

10. Utilisation d'au moins deux mélanges effervescents, solubles et /ou désintégrables différentes sous forme de comprimés ou de granulés, dont chacun contient une substance active différente ou une combinaison de substances actives différente, pour la préparation d'une solution ou suspension aqueuse unique d'un sel de calcium avec au moins un sel de fluor et/ou un diphosphonate pour le traitement de l'ostéoporose.

11. Utilisation d'au moins deux mélanges effervescents, solubles et/ou désintégrables différents sous forme de comprimés ou de granulés, dont chacun contient une substance active différente ou une combinaison de substances actives différente, pour la préparation d'une solution ou suspension aqueuse unique d'un sel de calcium avec au moins une préparation d'oestrogènes pour la prophylaxie de l'ostéoporose.
